# EUROPÄISCHE PATENTSCHRIFT

(11) **EP 0 939 070 B1**
(45) Veröffentlichungstag und Bekanntmachung des Hinweises auf die Patenterteilung: **23.06.2004**
(21) Anmeldenummer: 99102929.9
(22) Anmeldetag: 13.02.1999
(51) Int. Cl.: C07C 13/20, C07C 5/02, B01J 23/38

(54) **Verfahren zur Herstellung von Sechsring-Carbocyclen**
Process for the preparation of six-ring carbocyclic compounds
Procédé pour la préparation de composés carbocycliques à six anneaux

(30) Priorität: 26.02.1998 DE 19807995
(43) Veröffentlichungstag der Anmeldung: 01.09.1999
(73) Patentinhaber: Bayer MaterialScience AG, 51368 Leverkusen (DE)
(72) Erfinder: Albach, Rolf William, Dr., 51061 Köln (DE); Jautelat, Manfred, Dr., 51399 Burscheid (DE)

(56) Entgegenhaltungen:
- EP-A- 0 668 257
- DE-A- 4 443 705
- J. BLUM ET AL: "Catalytic hydrogenation of olefins, acetylenes and arenes by rhodium trichloride ans Aliquat-336 under hase transfer conditions" TETRAHEDRON LETTERS,1983, Seiten 4139-4142, XP002102978
- 'Roemmp (Katalyse) Fachgebiet Chemie, Unterthema Chemische Reaktionstechnik', 2003, GEORG THIEME VERLAG

## Beschreibung

Die vorliegende Erfindung betrifft ein Verfahren zur Herstellung von Sechsring-Carbocyclen durch Kernhydrierung von aromatischen Verbindungen mit Wasserstoff, wobei in einem Reaktionssystem aus zwei flüssigen, nicht miteinander mischbaren Phasen gearbeitet wird, dessen erste Phase durch die aromatische Verbindung und, falls erforderlich, ein mit Wasser nicht mischbares Lösungsmittel gebildet wird und die zweite Phase aus Wasser, einem darin kolloidal verteilten hydrieraktiven Metall als Hydrierkatalysator und Hilfsmitteln für die Stabilisierung des kolloidalen Katalysators besteht. Sechsring-Carbocyclen der erfindungsgemäß herstellbaren Art sind fachmännisch bekannte Zwischenprodukte zur Herstellung von Wirkstoffen und Industriechemikalien.

Es ist bereits bekannt, Sechsring-Carbocyclen durch Kernhydrierung von aromatischen Verbindungen herzustellen. Die Hydrierung wird im allgemeinen mit katalytisch angeregtem Wasserstoff durchgeführt; die hierfür geeigneten hydrieraktiven Metalle sind bekannt und sind im allgemeinen solche aus der Platinmetallgruppe sowie Nickel, Chrom, Niob oder Kupfer. Katalytische Hydrierungen in homogener Phase sind zwar durch geringe Diffusionshemmung, aber auch durch die Schwierigkeit gekennzeichnet, das Reaktionsprodukt durch Destillation, Kristallisation oder andere Verfahren vom Reaktionsgemisch und damit vom homogen gelösten Katalysator abzutrennen. Hierbei leidet gleichzeitig häufig die Aktivität des Hydrierkatalysators. Durch das Arbeiten mit aufgeschlämmtem Katalysator in der Slurry-Phase kann die Schwierigkeit der Abtrennung teilweise umgangen werden, obwohl immer noch die Notwendigkeit einer Grob- und einer Feinfiltration bleibt. Es bleibt jedoch die Gefahr der Desaktivierung des Katalysators, und es entsteht die neue Schwierigkeit der Erosion seitens des abzutrennenden Katalysators in Pumpen, Rohrleitungen, Ventilen und anderen Teilen der Apparatur. Schwierigkeiten der zuletzt genannten Art werden dadurch umgangen, daß man den Katalysator fest angeordnet im Reaktionsapparat anbringt und das Reaktionsgut in der Flüssigphase (z.B. Rieselphase) oder in der Gasphase über den Katalysator strömen läßt. Diese zu hoher Vollkommenheit gebrachte Reaktionsweise ist jedoch nur für Reaktionen mit großen Durchsätzen sinnvoll anzuwenden. Bei Produkten, die in kleinen Mengen und häufig nur chargenweise hergestellt werden, ist die Reaktionsführung unter Benutzung fest angeordneter Katalysatoren zu aufwendig.

Aus DE-A 44 43 705 ist bekannt, Benzol an einem mit Sulfobetain stabilisierten, kolloidalen Katalysator aus Ru auf La₂O₃-Träger zu Cyclohexen zu hydrieren; bei 50 bar und 150°C wurden in 0,5 Std. nur 8,5 % Umsatz und 78,5 % Selektivität erreicht. Nach Tetrahedron Letters 1983, 4139-42, verschlechtern sich die Hydrierergebnisse stark bei substituierten Benzolen. Aus WO 96/08462 ist die katalytische Hydrierung von aromatischen Aminen zu Cyclohexylaminen mit Wasserstoff an einem Ru/Al₂O₃-Katalysator in Gegenwart von LiOH, das die Desaminierung verhindern soll, bekannt; das Reaktionsmedium ist ein wasserhaltiges mit Wasser mischbares organisches Lösungsmittel.

Es wurde nun gefunden, daß es möglich ist, Sechsring-Carbocyclen durch Kemhydrierung der korrespondierenden aromatischen Verbindungen mit in verschiedener Phase angeordnetem Katalysator herzustellen, wobei das Reaktionsgut einerseits und der Hydrierkatalysator in kolloidaler Form andererseits in zwei nicht miteinander mischbaren flüssigen Phasen angeordnet sind. Das erfindungsgemäße Verfahren eignet sich hervorragend zur absatzweisen Durchführung von Kernhydrierungen aromatischer Verbindungen, es kann jedoch auch in kontinuierlicher Weise durchgeführt werden, wobei in einer dem Fachmann bekannten Mixer/Settler-Verfahrensweise vorgegangen wird.

Die Erfindung betrifft ein Verfahren zur Herstellung von Sechsring-Carbocyclen der Formel (II) durch Kernhydrierung von aromatischen Verbindungen der Formel (I) 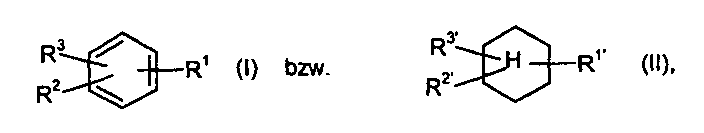 wobei in den Formeln
- R¹, R² und R³: unabhängig voneinander Wasserstoff, geradkettiges oder verzweigtes C₁-C₁₈-Alkyl, geradkettiges oder verzweigtes C₂-C₁₈-Alkenyl, C₃-C₈-Cycloalkyl, geradkettiges oder verzweigtes C₁-C₁₈-Alkoxy, Amino, Nitro, Hydroxy, Fluor, Cyano, -(C₀-C₄-Alkandiyl)-COO-(C₀-C₄-alkyl), -(C₂-C₄-Alkendiyl)-COO-(C₀-C₄-alkyl), -(C₀-C₄-Alkandiyl)-(C₆-C₁₂-aryl), -O-CO-(C₁-C₁₈-Alkyl), -O-(C₆-C₁₂-Aryl), -CO-(C₁-C₁₈-Alkyl), -CO-(C₆-C₁₂-Aryl), - S-(C₆-C₁₂-Aryl), -NH-(C₁-C₁₈-Alkyl), -N(C₁-C₁₈-Alkyl)(C₁-C₄-alkyl), -H-(C₆-C₁₂-Aryl), -N(C₆-C₁₂-Aryl)(C₁-C₄-alkyl) oder -P(X)ₘ(Y)ₙ(Z)_{q}(=O)ᵣ, worin X, Y und Z unabhängig voneinander für Phenyl oder C₁-C₄-Alkyl stehen und Y und Z zusätzlich und unabhängig voneinander OR⁴ oder NR⁴₂ bedeuten können, worin R⁴ Phenyl, C₁-C₄-Alkoxy oder C₁-C₄-Alkyl bedeutet und NR⁴₂ auch Piperidino darstellen kann, und Y und Z gemeinsam auch -O-CH₂CH₂-O- bedeuten können und der Klammerausdruck doppelt gebundenen Sauerstoff darstellt, m für die Zahl Null, Eins oder Zwei und n, q und r unabhängig voneinander für die Zahl Null oder Eins stehen, wobei die Summe m + n + q + r der Valenz von Phosphor entspricht, bedeuten, wobei Aryl-Bestandteile ein- oder zweifach durch C₁-C₄-Alkyl, C₁-C₄-Alkoxy, Amino, Nitro, Hydroxy oder verschiedene von ihnen substituiert sein können, und wobei weiterhin zwei der Reste R¹, R² und R³, wenn sie benachbart sind, gemeinsam den Rest eines ankondensierten Benzol- oder 1,2-Naphthalin- oder 2,3-Naphthalin-Systems, Trimethylen, Tetramethylen oder Propendiyl bedeuten können, die wie die obigen Aryl-Bestandteile substituiert sein können, und
- R^{1'}, R^{2'} und R³': unabhängig voneinander die Bedeutung von R¹, R² und R³ annehmen mit der Maßgabe, daß aus olefinisch ungesättigten Bestandteilen in R¹, R² bzw. R³ die korrespondierenden gesättigten Bestandteile, aus Nitro Amino, aus CO CHOH oder CH₂ und aus Cyano Aminomethyl entstehen können,
das dadurch gekennzeichnet ist, daß die Hydrierung bei 50 bis 180°C und bar bis 400 bar in einem Reaktionssystem aus zwei flüssigen, nicht miteinander mischbaren Phasen durchgeführt wird, in denen elementarer Wasserstoff dispergiert wird, wobei die erste Phase durch die aromatische Verbindung und, falls erforderlich, ein mit Wasser nicht mischbares Lösungsmittel gebildet wird, und die zweite Phase aus Wasser, einem darin kolloidal verteilten hydrieraktiven Metall der Gruppen Ib und VIII des Periodensystems der Elemente (Mendelejew) als Hydrierkatalysator und Hilfsmitteln für die Stabilisierung des kolloidalen Katalysators besteht und in Gegenwart von 100 bis 2 000 % der stöchiometrisch notwendigen Wasserstoffmenge gearbeitet wird.

Geradkettiges oder verzweigtes C₁-C₁₈-Alkyl ist beispielsweise Methyl, Ethyl, Propyl, Isopropyl, Butyl, Isobutyl, die isomeren Pentyle, Hexyle, Heptyle, Octyle, Decyle, Dodecyle, Hexadecyle und Octadecyle. Geradkettiges oder verzweigtes C₂-C₁₈-Alkenyl leitet sich vom korrespondierenden Alkyl dadurch ab, daß eine olefinische Doppelbindung vorliegt; sie kann innenständig oder endständig sein. Geradkettiges oder verzweigtes C₁-C₁₈-Alkoxy leitet sich vom korrespondierenden Alkyl dadurch ab, daß das Alkyl über ein Ether-Sauerstoffatom gebunden ist.

In bevorzugter Weise haben Alkyl bzw. Alkoxy 1 bis 12 C-Atome und Alkenyl 2 bis 12 C-Atome. In besonders bevorzugter Weise haben Alkyl bzw. Alkoxy 1 bis 4 C-Atome; Alkenyl hat besonders bevorzugt 2 bis 4 C-Atome.

Cycloalkyl ist beispielsweise Cyclopropyl, Cyclobutyl, Cyclopentyl, Methyl-cyclopentyl, Dimethyl-cyclopentyl, Cyclohexyl, Methyl-cyclohexyl, Dimethyl-cyclohexyl, Cycloheptyl oder Cyclooctyl. In bevorzugter Weise stellt Cycloalkyl Cyclopropyl, Cyclopentyl oder Cyclohexyl dar.

Die Gruppe C₀-C₄-Alkandiyl stellt eine Spacer-Gruppe dar und ist demnach zweibindig. Sie kann jedoch zu einer Einfachbindung degenerieren, was durch den Ausdruck C₀ (kein C-Atom) gekennzeichnet wird. In ganz analoger Weise stellt die Gruppe C₀-C₄-Alkyl den alkoholischen Teil einer Estergruppe dar, wobei im Falle C₀ die Estergruppe zum Säure-Wasserstoffatom (kein C-Atom) degeneriert. Die Gruppe -(C₀-C₄-Alkandiyl)-COO-(C₀-C₄-Alkyl) kann somit die nicht veresterte Carboxylgruppe -COOH darstellen, wenn in beiden Klammerausdrücken C₀ vorliegt. Für den Fall, daß in beiden Klammern C₄ vorliegt, handelt es sich um den Butylester der an den Kern der aromatischen Verbindung (I) gebundenen Valeriansäure. Die dazwischenliegenden homologen Substituenten sind dem Fachmann bekannt.

Die Gruppe -(C₂-C₄-Alkendiyl)-COO-(C₀-C₄-Alkyl) umfaßt z.B. für den Fall eines C₂-Alkendiyls die Zimtsäure bzw. ihre Ester; weitere Homologe dieser Gruppe sind dem Fachmann bekannt.

Einzelsubstituenten der Gruppe -(C₀-C₄-Alkandiyl)-(C₆-C₁₂-aryl) umfassen für den Fall von C₀ nichtgespacerte Arylsubstituenten, wie Phenyl, Naphthyl oder Biphenylyl und beispielsweise für den Fall C₁ Benzyl, für den Fall C₂ β-Phenylethyl usw. Die Gruppen -O-(C₆-C₁₂-Aryl) und -S-(C₆-C₁₂-Aryl) umfassen beispielsweise Phenoxy, Naphthyloxy, Biphenyloxy und die entsprechenden Thioanaloga.

Die Substituenten R¹, R² und R³ können weiterhin die im einzelnen angegebenen primären bzw. sekundären Aminogruppen mit Alkyl- bzw. Arylresten darstellen.

R¹, R² und R³ können ferner Phenyl- bzw. Alkylphosphan bzw. das daraus hervorgehende Phosphinoxid darstellen.

Die in den Substituenten enthaltenen Aryl-Bestandteile können weiterhin ein- oder zweifach durch C₁-C₄-Alkyl, C₁-C₄-Alkoxy, Amino, Nitro. Hydroxy oder verschiedene von ihnen substituiert sein.

Im Falle der obengenannten ankondensierten Systeme gelangt man zum Naphthalin(benzokondensiert) als aromatischer Verbindung und in analoger Weise zum Indan-System, Tetrahydronaphthalin-System oder Inden-System. Auch diese ankondensierten Teile, die zu den genannten Systemen fuhren, können wie die obengenannten Aryl-Bestandteile substituiert sein.

Die Substituenten des Sechsring-Carbocyclus (II), nämlich R^{1'}, R^{2'} und R^{3'}, unterscheiden sich von R¹, R² und R³ lediglich darin, daß Substituenten, die bei der Hydrierung des aromatischen Kerns gleichfalls mithydriert werden, in die entsprechenden hydrierten Spezies umgewandelt werden. Beispielsweise können olefinisch ungesättigten Teile in die zugehörigen gesättigten Teile umgewandelt werden; so kann beispielsweise aus der Zimtsäure die β-Phenylpropionsäure werden. Weiterhin kann eine Nitrogruppe in die Aminogruppe, eine Carbonylgruppe in eine Methylengruppe und die Cyanogruppe in Aminomethyl umgewandelt werden. Im Falle der Umwandlung einer Nitrogruppe in eine Aminogruppe oder für den Fall, daß bereits eine Aminogruppe vorliegt, kann es bei der Kernhydrierung und der gegebenenfalls simultan erfolgenden Hydrierung der Nitrogruppe zu einer weiteren Reaktion an der Aminogruppe und der Bildung von Dicyclohexylamin kommen. Diese Reaktion kann in bekannter Weise über Zwischenstufen erfolgen, beispielsweise über C^{y}clohexenylamin, Cyclohexanimin oder N-Phenyl-Cyclohexylamin. Solche Zwischenstufen und ihre weitere Reaktion sind dem Fachmann bekannt.

In bevorzugter Weise werden aromatische Verbindungen der Formel 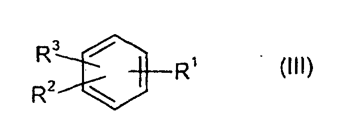 eingesetzt, worin
- R¹, R² und R³: unabhängig voneinander Wasserstoff, geradkettiges oder verzweigtes C₁-C₁₈-Alkyl, C₃-, C₅- oder C₆-Cycloalkyl, geradkettiges oder verzweigtes C₁-C₁₈-Alkoxy, Amino, Nitro, Hydroxy, -(C₀-C₄-Alkandiyl)-COO-(C₀-C₄-alkyl), -CH=CH-COO-(C₀-C₄-Alkyl), -(C₀-C₄-Alkandiyl)-phenyl, -O-Phenyl, -S-Phenyl, -NH-(C₁-C₁₈-Alkyl), -N(C₁-C₁₈-Alkyl)(C₁-C₄-alkyl), -NH-Phenyl oder -N-(Phenyl)(C₁-C₄-Alkyl) bedeuten, wobei Phenyl-Bestandteile ein- oder zweifach durch C₁-C₄-Alkyl, C₁-C₄-Alkoxy, Amino, Nitro, Hydroxy oder verschiedene von ihnen substituiert sein können, und wobei weiterhin zwei der Reste R¹, R² und R³, wenn sie benachbart sind, gemeinsam den Rest eines ankondensierten Benzol-Systems, Trimethylen oder Tetramethylen bedeuten können, die wie die obigen Phenyl-Bestandteile substituiert sein können.

In besonders bevorzugter Weise werden im erfindungsgemäßen Verfahren aromatische Verbindungen der Formel 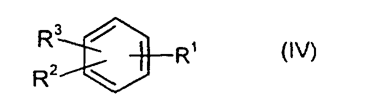 eingesetzt, worin
- R¹: dieselbe Bedeutung wie in Formel (III) besitzt.
- R²: für Wasserstoff, C₁-C₄-Alkyl, C₁-C₄-Alkoxy, Amino, Nitro oder Hydroxy steht und
- R³: Wasserstoff C₁-C₄-Alkyl oder C₁-C₄-Alkoxy bedeutet, wobei weiterhin die Reste R² und R³, wenn sie benachbart sind, gemeinsam den Rest eines ankondensierten Benzol-Systems, Trimethylen oder Tetramethylen bedeuten können, die wie die Phenyl-Bestandteile von R¹ substituiert sein können.

Beispiele für aromatische Verbindungen, die erfindungsgemäß eingesetzt werden können, sind: Benzol, Toluol, Xylol, Ethylbenzol, Palmitylbenzol, Stearylbenzol, Cumol, Cyclopropyl-benzol, Cyclohexyl-benzol, Styrol, Butenylbenzol, Dodecenylbenzol, Anisol, Phenyl-ethylether, Phenyl-butylether, Phenyl-stearylether, Anilin, Phenylendiamin, Nitrobenzol, Dinitrotoluol, Phenol, Resorcin, Brenzkatechin, Fluorbenzol, Difluorbenzol, Trifluorbenzol, Benzonitril, Dicyanobenzol, Benzoesäure, Benzoesäuremethylester, Benzoesäurebutylester, Phenylessigsäure, Phenylessigsäuremethylester, β-Phenyl-propionsäure, β-Phenyl-propionsäuremethylester, Zimtsäure, Zimtsäuremethylester, Zimtsäurebutylester, Biphenyl, Diphenylmethan, 1,2-Diphenyl-ethan, 2,2-Diphenyl-propan, Bisphenol-A, Benzophenon, Acetophenon, Essigsäurephenylester, Propionsäurephenylester, Buttersäurephenylester, Diphenylether, Naphthylphenylether, Diphenylthioether, N-Methyl-anilin, N-Ethyl-anilin, N-Stearyl-anilin, N-Stearyl-N-methylanilin, Diphenylamin, N-Naphthyl-anilin, N-Methyldiphenylamin, Triphenylphosphan, Triphenylphosphinoxid, Diphenylmethyl-phosphan. Phenyl-methyl-phosphan, Diphenylmethyl-phosphinoxid, Phenyl-methylphosphinoxid (oder seine tautomere Form C₆H₅-P(CH₃)-OH), Phenyl-di-piperidinophosphan, Phenyl-di-methoxy-phosphinoxid, P-Phenyl-1-phospha-2,5-dioxa-cyclopentan, 2,4-Dinitro-toluol, 4,4'-Diamino-diphenyl-methan, 2,6-Dinitro-toluol, 2,4'-Diamino-diphenyl-methan.

Die hieraus entstehenden Sechsring-Carbocyclen sind dem Fachmann bekannt und im Lichte der oben gegebenen Erläuterungen zweifelsfrei zuzuordnen.

Das erfindungsgemäße Verfahren ist gekennzeichnet durch seine Durchführung in einem Reaktionssystem aus zwei flüssigen, nicht miteinander mischbaren Phasen. Die erste dieser beiden Phasen wird durch die zu hydrierende aromatische Verbindun^{g} im Gemisch mit der im Verlaufe der Reaktion entstehenden carbocyclischen Verbindung gebildet. Somit sind also in erster Linie flüssige aromatische Verbindungen dem erfindungsgemäßen Verfahren zugänglich, aus denen wiederum flüssige Sechsring-Carbocyclen gebildet werden. Das erfindungsgemäße Verfahren ist jedoch weiterhin auch festen aromatischen Verbindungen zugänglich, wenn ein geeignetes hydrierinertes Lösungsmittel eingesetzt wird. Hierfür verwendbare Lösungsmittel sollen mit der zweiten, im wesentlichen wäßrigen Phase nicht oder nur unwesentlich mischbar sein. Geeignete Lösungsmittel dieser Art sind beispielsweise C₅-C₁₂-Alkane oder C₅-C₈-Cycloalkane, wie Pentan, Cyclopentan, Hexan, Cyclohexan, Petrolether, Benzinfraktionen und andere Kohlenwasserstoffmischungen. Weitere geeignete Lösungsmittel sind die mit Wasser nur begrenzt mischbaren höheren Alkohole mit 5 bis 10 C-Atomen, wie die geradkettigen oder verzweigten Pentanole, Hexanole, Octanole oder Decanole.

Weitere geeignete Lösungsmittel sind CO₂, das sich unter den erfindungsgemäßen Bedingungen auch im überkritischen Zustand befinden kann, Ether, wie Diisopropylether, Tetrahydrofuran oder Dioxan, sowie Halogenalkane, wie Methylenchlorid oder Dichlorethan und perfluorierte bzw. zu > 90 % fluorierte Alkane. Für den Fall der Mitverwendung solcher Lösungsmittel beträgt ihre Menge 10 bis 1 000 Gew.-%, bezogen auf das Gewicht der zu hydrierenden aromatischen Verbindung, bevorzugt 20 bis 500 Gew.-%.

Die zweite Phase des Reaktionssystems besteht im wesentlichen aus Wasser und enthält darin kolloidal verteiltes hydrieraktives Metall als Hydrierkatalysator. Die zweite Phase enthält ferner Stoffe, die geeignet sind, das kolloidal verteilte hydrieraktive Metall zu stabilisieren.

Hydrieraktive Metalle, die für das erfindungsgemäße Verfahren geeignet sind und sich kolloidal verteilen lassen, sind beispielsweise solche der Gruppen Ib und VIII des Periodensystems der Elemente (Mendelejew). Als Einzelvertreter seien beispielsweise genannt: Silber, Gold, Nickel, Palladium, Ruthenium, Rhodium, Platin, Iridium, bevorzugt Palladium, Ruthenium, Rhodium, Platin und Nickel. Diese Metalle können sowohl einzeln, als auch im Gemisch mehrerer von ihnen eingesetzt werden. Bevorzugte Gemische sind Pd/Ru, Pd/Rh, Ni/Ru und Ni/Rh. Für den Fall solcher Zweiergemische beträgt das Gewichtsverhältnis der beteiligten Metalle darin 99:1 bis 1:99. Die hydrieraktiven Metalle, einzeln oder als Gemisch, sind im gesamten Reaktionssystem in einer solchen Menge vorhanden, daß zu Beginn der Reaktion 50 bis 5 000 mol der zu hydrierenden aromatischen Verbindung pro g-Atom hydrieraktiven Metalls vorliegen. In einer weiteren Ausführungsform des erfindungsgemäßen Verfahrens können 1 bis 50 Atom-% des hydrieraktiven Metalls durch Zinn in elementarer oder ionischer Form ersetzt sein.

Das erfindungsgemäße Verfahren wird bei einer Temperatur von 50 bis 100°C, bevorzugt 60 bis 80°C und einem Druck von 1 bis 400 bar, bevorzugt 10 bis 400 bar, und insbesondere von 20 bis 150 bar, durchgeführt. Der Druck über dem Reaktionssystem wird durch den vorhandenen elementaren Wasserstoff oder durch ein Gemisch aus Wasserstoff und einem inerten Gas aufrecht erhalten; solche inerten Gase sind beispielsweise Stickstoff, Argon, Neon oder ein Gemisch mehrerer von ihnen. In jedem Falle liegt eine Wasserstoffmenge vor, die 100 bis 2000 % der stöchiometrisch zur oben beschriebenen Hydrierung notwendigen H₂-Menge, bevorzugt 150 bis 1 500 %, besonders bevorzugt 200 bis 1 000 %, beträgt.

Zur Stabilisierung der kolloidal verteilten hydrieraktiven Metalle in der wäßrigen Phase, enthält diese wäßrige Phase zusätzlich Stabilisatoren, wie Tenside aus der Gruppe der anionischen und kationischen Tenside, der amphiphilen Tenside, wie Betaine und Sulfobetaine, sowie amphiphile Zuckertenside, und der nichtionischen Tenside, wie Polyalkylenether, unter anderem von Fettalkoholen und monoveresterten Kohlenhydraten. Solche Tenside sind dem Fachmann bekannt.

Weitere Zusätze sind basisch reagierende Salze, z.B. LiOH, Li₂CO₃, NaOH, Na-Acetat, Kaliumphosphat, Natriumhydrogenphosphat und ähnliche.

Das erfindungsgemäße Verfahren kann absatzweise oder kontinuierlich durchgeführt werden. Bei der absatzweisen Durchführung wird das Reaktionsgemisch, bestehend aus der ersten Phase (organische Phase mit dem Substrat) und der zweiten Phase (wäßrige Phase mit dem kolloidal verteilten Metall), in einem Autoklaven mit einer Misch- oder Rühreinrichtung unter sauerstofffreien Bedingungen mit Wasserstoff oder einer Mischung H₂/Inertgas unter den gewählten Reaktionsdruck versetzt und auf die gewählte Reaktionstemperatur erwärmt. Wasserstoff kann im Maße seines Verbrauchs nachgesetzt werden; alternativ hierzu kann bei einem erhöhten Wasserstoffdruck begonnen werden und die Reaktion dann beendet werden, wenn kein weiterer Druckabfall beobachtet wird, wobei man im Rahmen des oben angegebenen Druckbereiches bleibt. Durch das Mischen bzw. Rühren der beiden Phasen des Reaktionssystems wird der vorhandene Hydrierwasserstoff darin dispergiert. Bei kontinuierlicher Reaktionsführung können die beiden flüssigen Phasen in einem geeigneten Reaktor, z.B. in einem Rohrreaktor, Überlaufrührkessel oder Kesselkaskade, im Gleich- oder Gegenstrom geführt werden, wobei an einer oder mehreren Stellen zugesetzter Wasserstoff ebenfalls dispergiert wird. Nach Beendigung der Reaktion werden die beiden Phasen, beispielsweise durch einfaches Absitzenlassen, getrennt. Die organische Phase wird zur Reindarstellung des Produkts und zur Rückgewinnung des gegebenenfalls nicht vollständig umgesetzten Edukts üblichen Trennungsmaßnahmen unterworfen, wie beispielsweise einer Destillation, Kristallisation oder präparativen Chromatographie. Die wäßrige Phase mit dem darin enthaltenen kolloidal verteilten metallischen Hydrierkatalysator kann in einen weiteren Ansatz des erfindungsgemäßen Verfahrens zurückgeführt werden.

### Beispiele

### I. Herstellung von hydrieraktiven Kolloiden

### Beispiel 1

887 mg PdCl₂ (5 mmol), 0,37 g Li₂CO₃ (5 mmol) und 6,71 g Sulfobetain der Formel C₁₂H₂₅N⁺(CH₃)₂(CH₂)₃SO₃- (SB12, 20 mmol) werden in 0,1 l von O₂ befreitem Wasser 3 Stunden bei Raumtemperatur unter Einleitung von Wasserstoff gerührt. Man erhält eine schwarze Lösung. Von der Lösung kann das Wasser am Rotationsverdampfer entfernt werden, wobei man ein schwarzes Wachs erhält. Dieses Wachs ist nach 1 Woche an Luft noch vollständig wasserlöslich. Die Elektronenmikroskopie zeigt ein sehr fein verteiltes Kolloid. Bei stärkerer Vergrößerung sieht man gut die Primärteilchen einer Größe von 2 bis 5 nm; das Atomverhältnis wird mit Pd:Cl:S ≈ 1:0,4:2 festgestellt.

### Beispiel 2

546 mg NiBr₂ (2,5 mmol), 0,185 g Li₂ CO₃ (2,5 mmol) und 3,36 g SB12 wie in Beispiel 1 (10,5 mmol) werden in 100 ml O₂-freiem Wasser suspendiert. Wasserstoff wird während einer Stunde eingeleitet, wobei sich eine Farbänderung von gelb nach hellgrün einstellt. Portionsweise werden 56,7 mg NaBH₄ (1,5 mmol) zugegeben. Daraufhin wird die Lösung innerhalb 1 Minute unter starkem Schäumen schwarz. Man läßt diese Lösung 2 Stunden weiter unter Rühren ausreagieren und setzt ein Aliquot für die Hydrierungen ein.

### Beispiel 3

0,56 g Pd-Acetat (2,5 mmol) werden in 10 ml Toluol vorgelegt. Hierzu werden 0,35 ml konzentrierter HNO₃ (5 mmol) zugegeben. Nach 1 Stunde wird der größte Teil des Toluols am Rotationsverdampfer entfernt. Der Rückstand wird in 100 ml Wasser (gesättigt mit Argon) aufgenommen; hierzu werden 666 mg RhCl₃ (2,5 mmol) und 6,71 g eines Sulfobetains analog Beispiel 1, jedoch nur mit einer C₈-Alkylkette am N-Atom (SB8) zugegeben. Zu dieser Lösung werden 0,19 g NaBH₄ portionsweise zugegeben; zur Vervollständigung der Reaktion läßt man 3 Stunden nachrühren. Die Elektronenmikroskopie zeigt ein Netzwerk von stark verdichteten Zentren und helleren Agglomeratbereichen (Atomverhältnis Rh:Pd ≈ 1 in beiden Bereichen). Bei stärkerer Vergrößerung erkennt man im vernetzten Bereich Primärteilchen.

### Beispiel 4

0,56 g Pd-Acetat (2,5 mmol) werden in 10 ml Toluol mit 0,35 ml konzentrierter HNO₃ (5 mmol) 1 Stunde gerührt. Das Toluol wird danach am Rotationsverdampfer weitgehend entfernt. Der Rückstand wird in 100 ml Wasser (gesättigt mit Argon) aufgenommen, und mit 666 mg RhCl₃ (2,5 mmol) und 6 g Polyoxyethylenlaurylether (5 mmol) versetzt. 0,19 g NaBH₄ werden portionsweise zugegeben. Man läßt zur Vervollständigung der Reaktion 3 Stunden nachrühren und erhält eine schwarze Lösung.

### Beispiel 5

444 mg PdCl₂ (2,5 mmol), 654 mg RuCl₃ (2,5 mmol), 369 mg Li₂CO₃ (5 mmol) und 6,71 g des Sulfobetains von Beispiel 1 (20 mmol) werden in 100 ml Wasser (gesättigt mit Argon) bei Raumtemperatur unter Wasserstoff-Atmosphäre gerührt. Man erhält eine schwarze Lösung, die beim Filtrieren nur sehr wenig Bodensatz ergibt. Die Elektronenmikroskopie zeigt ein Netzwerk von stark verdichteten Zentren (sowohl Ru:Pd ≈ 2, als auch Ru:Pd ≈ 0,3) und helleren Agglomeratbereichen (Ru:Pd ≈ 1). Bei stärkerer Vergrößerung sieht man im Agglomeratbereich gut die Primärteilchen mit Abmessungen von 4 bis 5 nm und einem Atomverhältnis Ru:Pd ≈ 1, aber daneben viele Drillinge mit Abmessungen von 10 bis 20 nm.

### II. Hydrierungen

### Beispiel 6

36 ml Isopropylbenzol (Cumol) und 25,8 ml der kolloidalen Lösung aus Beispiel 5 (0,05 mol/l, 1,29 mmol) werden in einem 0,1 l-VA-Autoklaven während 15 Stunden bei 150°C und 60 bar H₂ gerührt. Das GCMS (EI) zeigt 99,5 % Isopropyl-cyclohexan.

### Beispiel 7

36 ml Cumol und 25,8 ml des in Beispiel 6 verwendeten Katalysator-Sols werden in einem 0,1 1-VA-Autoklaven während 15 Stunden bei 100°C und 60 bar H₂ gerührt. Nach dem Entspannen wird die organische Phase mit Methylenchlorid verdünnt und abgetrennt. Sowohl ¹H-NMR als auch GCMS (EI) zeigen 100 % gebildetes Isopropyl-cyclohexan an. Die abgetrennte wäßrige Phase wird erneut mit 36 ml Cumol versetzt und während 15 Stunden bei 100°C und 60 bar H₂ erneut gerührt. Nach dem Entspannen wird die organische Phase mit Methylenchlorid verdünnt und abgetrennt. Das ¹HNMR-Spektrum zeigt erneut 100 % Isopropyl-cyclohexan an.

### Beispiel 8

22,9 ml Benzol und 25,6 ml des in Beispiel 6 verwendeten Katalysator-Sols (0,05 mol/l, 1,29 mmol) werden in einem 0,1 l-VA-Autoklaven während 15 Stunden bei 100°C und 60 bar H₂ gerührt. Nach dem Entspannen und Dekantieren erhält man Cyclohexan mit einer Reinheit von 99,89 % (GC). Die wäßrige Phase wird zu 97,4 % wiedergewonnen.

### Beispiel 9

5,1 g 4,4'-Diamino-diphenylmethan wurden in 25 ml 1-Pentanol gelöst. 5,12 ml des Katalysatorsols nach Beispiel 5 werden dazugegeben, und das Reaktionsgemisch wird in einem 0,1 l-Autoklaven während 15 Std. bei 100°C und 60 bar H₂ hydriert. Man erhält bei 100 % Umsatz 4,4'-Diamino-biscyclohexylmethan mit einer Selektivität von 32,1 % und nur teilweise kernhydriertes 4-(4'-Amino-cycloxyl)-anilin mit einer Selektivität von 12,4 %. Offenbar infolge NH₂-OH-Austauschs wegen der Anwesenheit von Wasser werden weiterhin 4-Amino-4'-hydroxy-biscyclohexyl (45,3 % Selektivität) und 4-(4'-Hydroxy-cyclohexyl)-anilin (4,2 % Selektivität) gefunden. Der Rest zu 100 % Selektivität besteht aus Nebenprodukten.

## Patentansprüche

1. Verfahren zur Herstellung von Sechsring-Carbocyclen der Formel (II) durch Kernhydrierung von aromatischen Verbindungen der Formel (I) 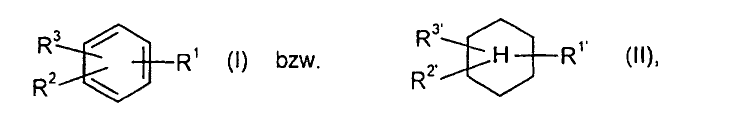 wobei in den Formeln
R¹, R² und R³ unabhängig voneinander Wasserstoff, geradkettiges oder verzweigtes C₁-C₁₈-Alkyl, geradkettiges oder verzweigtes C₂-C₈-Alkenyl, C₃-C₈-Cycloalkyl, geradkettiges oder verzweigtes C₁-C₁₈-Alkoxy, Amino, Nitro, Hydroxy, Fluor, Cyano, -(C₀-C₄-Alkandiyl)-COO-(C₀-C₄-alkyl), -(C₂-C₄-Alkendiyl)-COO-(C₀-C₄-alkyl), -(C₀-C₄-Alkandiyl)-(C₆-C₁₂-aryl), -O-CO-(C₁-C₁₈-Alkyl), -O-(C₆-C₁₂-Aryl), -CO-(C₁-C₁₈-Alkyl), -CO-(C₆-C₁₂-Aryl, -S-(C₆-C₁₂Aryl), -NH-(C₁-C₁₈-Alkyl), -N(C₁-C₁₈-Alkyl)(C₁-C₄-alkyl), -NH-(C₆-C₁₂-Aryl), -N(C₆-C₁₂-Aryl)(C₁-C₄-alkyl) oder -P(X)ₘ(Y)ₙ(Z)_{q}(=O)ᵣ, worin X, Y und Z unabhängig voneinander für Phenyl oder C₁-C₄-Alkyl stehen und Y und Z zusätzlich und unabhängig voneinander OR⁴ oder NR⁴ bedeuten können, worin R⁴ Phenyl, C₁-C₄-Alkoxy oder C₁-C₄-Alkyl bedeutet und NR⁴₂ auch Piperidino darstellen kann, und Y und Z gemeinsam auch -O-CH₂CH₂-O- bedeuten können und der Klammerausdruck doppelt gebundenen Sauerstoff darstellt, m für die Zahl Null, Eins oder Zwei und n, q und r unabhängig voneinander für die Zahl Null oder Eins stehen, wobei die Summe m + n + q +r der Valenz von Phosphor entspricht, bedeuten, wobei Aryl-Bestandteile ein- oder zweifach durch C₁-C₄-Alkyl, C₁-C₄-Alkoxy, Amino. Nitro, Hydroxy oder verschiedene von ihnen substituiert sein können, und wobei weiterhin zwei der Reste R¹, R² und R³, wenn sie benachbart sind, gemeinsam den Rest eines ankondensierten Benzol- oder 1,2-Naphthalin- oder 2,3-Naphthalin-Systems, Trimethylen, Tetramethylen oder Propendiyl bedeuten können, die wie die obigen Aryl-Bestandteile substituiert sein können, und
R^{1'}, R^{2'} und R^{3'} unabhängig voneinander die Bedeutung von R¹, R² und R³ annehmen mit der Maßgabe, daß aus olefinisch ungesättigten Bestandteilen in R¹, R² bzw. R³ die korrespondierenden gesättigten Bestandteile, aus Nitro Amino, aus CO CHOH oder CH₂ und aus Cyano Aminomethyl entstehen können,
**dadurch gekennzeichnet, daß** die Hydrierung bei 50 bis 180°C und 1 bar bis 400 bar in einem Reaktionssystem aus zwei flüssigen, nicht miteinander mischbaren Phasen durchgeführt wird, in denen elementarer Wasserstoff dispergiert wird, wobei die erste Phase durch die aromatische Verbindung und, falls erforderlich, ein mit Wasser nicht mischbares Lösungsmittel gebildet wird und die zweite Phase aus Wasser, mindestens einem darin kolloidal verteilten hydrieraktiven Metall der Gruppen Ib und VIII des Periodensystems der Elemente (Mendelejew) als Hydrierkatalysator und Hilfsmittel für die Stabilisierung des kolloidalen Katalysators besteht und in Gegenwart von 100 bis 2 000 % der stöchiometrisch notwendigen Wasserstoffmenge gearbeitet wird.

2. Verfahren nach Anspruch 1, **dadurch gekennzeichnet, daß** aromatische Verbindungen der Formel 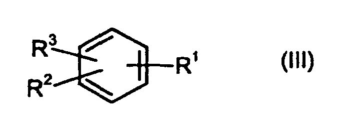 eingesetzt, worin
R¹, R² und R³ unabhängig voneinander Wasserstoff, geradkettiges oder verzweigtes C₁-C₁₈-Alkyl, C₃-, C₅- oder C₆-Cycloalkyl, geradkettiges oder verzweigtes C₁-C₁₈-Alkoxy, Amino, Nitro, Hydroxy, -(C₀-C₄-Alkandiyl)-COO-(C₀-C₄-alkyl), -CH=CH-COO-(C₀-C₄-Alkyl), -(C₀-C₄-Alkandiyl)-phenyl, -O-Phenyl, -S-Phenyl, -NH-(C₁-C₁₈-Alkyl), - N(C₁-C₁₈-Alkyl)(C₁-C₄-alkyl), -NH-Phenyl oder -N-(Phenyl)(C₁-C₄-Alkyl) bedeuten, wobei Phenyl-Bestandteile ein- oder zweifach durch C₁-C₄-Alkyl, C₁-C₄-Alkoxy, Amino, Nitro, Hydroxy oder verschiedene von ihnen substituiert sein können, und wobei weiterhin zwei der Reste R¹, R² und R³, wenn sie benachbart sind, gemeinsam den Rest eines ankondensierten Benzol-Systems, Trimethylen oder Tetramethylen bedeuten können, die wie die obigen Phenyl-Bestandteile substituiert sein können.

3. Verfahren nach Anspruch 2, **dadurch gekennzeichnet, daß** aromatische Verbindungen der Formel 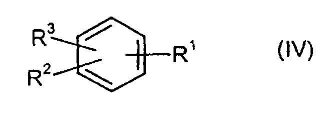 eingesetzt werden, worin
R¹ die in Anspruch 2 angegebene Bedeutung hat,
R² für Wasserstoff, C₁-C₄-Alkyl, C₁-C₄-Alkoxy, Amino, Nitro oder Hydroxy steht und
R³ Wasserstoff. C₁-C₄-Alkyl oder C₁-C₄-Alkoxy bedeutet, wobei weiterhin die Reste R² und R³, wenn sie benachbart sind, gemeinsam den Rest eines ankondensierten Benzol-Systems, Trimethylen oder tetramethylen bedeuten können, die wie die Phenyl-Bestandteile von R¹ substituiert sein können.

4. Verfahren nach einem oder mehreren der Ansprüche 1 bis 3, **dadurch gekennzeichnet, daß** die Hydrierung bei 60 bis 80°C durchgeführt wird.

5. Verfahren nach einem oder mehreren der Ansprüche 1 bis 4, **dadurch gekennzeichnet, daß** die Hydrierung bei einem Druck von 10 bis 400, bevorzugt von 20 bis 150 bar durchgeführt wird.

6. Verfahren nach einem oder mehreren der Ansprüche 1 bis 5, **dadurch gekennzeichnet, daß** in Gegenwart von 150 bis 1 500 %, bevorzugt 200 bis 1 000 %, der zur Hydrierung stöchiometrisch notwendigen Wasserstoffmenge gearbeitet wird.

7. Verfahren nach einem oder mehreren der Ansprüche 1 bis 6, **dadurch gekennzeichnet, daß** das kolloidal verteilt Metall als Hydrierkatalysator ein Einzelmetall oder eine Metallkombination aus der Gruppe von Pd, Ru, Rh, Pt, Ni, Pd/Ru, Pd/Rh, Ni/Ru und Ni/Rh ist.

8. Verfahren nach einem oder mehreren der Ansprüche 1 bis 7, **dadurch gekennzeichnet, daß** 1 bis 50 Atom-% des hydrieraktiven Metalls durch Zinn in metallischer oder ionischer Form ersetzt ist.

9. Verfahren nach einem oder mehreren der Ansprüche 1 bis 8, **dadurch gekennzeichnet, daß** für den Fall, daß ein Lösungsmittel zur Bildung der ersten Phase mit der zu hydrierenden aromatischen Verbindung erforderlich ist, eines aus der Gruppe der C₅-C₁₂-Alkane, C₅-C₈-Cycloalkane, der C₅-C₁₀-Alkanole, CO₂, der offenkettigen und cyclischen C₄-C₈-Ether und Methylenchlorid in einer Menge von 10 bis 1 000 Gew.-%, bezogen auf das Gewicht der aromatischen Verbindung, eingesetzt wird.

10. Verfahren nach Anspruch 9, **dadurch gekennzeichnet, daß** die Menge des eingesetzten Lösungsmittels 20 bis 500 Gew.-%, bezogen auf das Gewicht der aromatischen Verbindung, beträgt.

## Claims

1. Process for the preparation of 6-membered ring carbocycles of the formula (II) by ring hydrogenation of aromatic compounds of the formula (I) 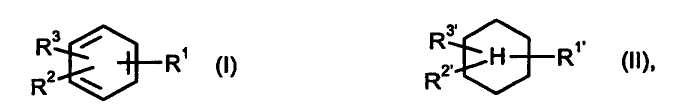 where, in the formulae,
R¹, R² and R³ independently of one another are hydrogen, straight-chain or branched C₁-C₁₈-alkyl, straight-chain or branched C₂-C₁₈-alkenyl, C₃-C₈-cycloalkyl, straight-chain or branched C₁-C₁₈-alkoxy, amino, nitro, hydroxyl, fluoro, cyano, -(C₀-C₄-alkanediyl)-COO-(C₀-C₄-alkyl), -(C₂-C₄-alkenediyl)-COO-(C₀-C₄-alkyl), -(C₀-C₄-alkanediyl)-C₆-C₁₂-aryl), - O-CO-(C₁-C₁₈-alkyl), -O-(C₆-C₁₂-aryl), -CO-(C₁-C₁₈-alkyl), -CO-(C₆-C₁₂-aryl), -S-(C₆-C₁₂-aryl), -NH-(C₁-C₁₈-alkyl), -N(C₁-C₁₈-alkyl)(C₁-C₄-alkyl), -NH-(C₆-C₁₂-aryl), -N(C₆-C₁₂-aryl)(C₁-C₄-alkyl) or - P(X)ₘ(Y)ₙ(Z)_{q}(=O)ᵣ, where X, Y and Z independently of one another are phenyl or C₁-C₄-alkyl, and Y and Z additionally and independently of one another may be OR⁴ or NR⁴₂, where R⁴ is phenyl, C₁-C₄-alkoxy or C₁-C₄-alkyl, and NR⁴₂ can also be piperidino, and Y and Z together can also be -O-CH₂CH₂-O-, and the bracketed term represents double-bonded oxygen, m is zero, one or two, and n, q and r independently of one another are zero or one, the sum m + n + q + r corresponding to the valency of phosphorus, where aryl constituents may be mono- or disubstituted by C₁-C₄-alkyl, C₁-C₄-alkoxy, amino, nitro, hydroxyl or mixtures thereof, and where in addition two of the radicals R¹, R² and R³, if they are adjacent, can together be the radical of a fused benzene or 1,2-naphthalene or 2,3-naphthalene system, trimethylene, tetramethylene or propenediyl, each of which may be substituted like the aryl constituents above, and
R^{1'}, R²' and R^{3'} independently of one another are as defined for R¹, R² and R³, with the proviso that olefinically unsaturated constituents in R¹, R² and R³ can form the corresponding saturated constituents, nitro can form amino, CO can form CHOH or CH₂ and cyano can form aminomethyl,
**characterized in that** the hydrogenation is carried out at from 50 to 180°C and from I bar to 400 bar in a reaction system consisting of two liquid, immiscible phases in which elemental hydrogen is dispersed, the first phase being formed by the aromatic compound and, if necessary, a water-immiscible solvent, and the second phase consisting of water, and, colloidally dispersed therein, at least one hydrogenation-active metal from groups Ib and VIII of the Periodic Table of the Elements (Mendeleev) as hydrogenation catalyst, and auxiliaries for stabilizing the colloidal catalyst, and in the presence of from 100 to 2000% of the stoichiometrically necessary amount of hydrogen.

2. Process according to Claim 1, **characterized in that** aromatic compounds of the formula 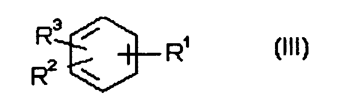 are used, in which
R¹, R² and R³ independently of one another are hydrogen, straight-chain or branched C₁-C₁₈-alkyl, C₃-, C₅- or C₆-cycloallcyl, straight-chain or branched C₁-C₁₈-alkoxy, amino, nitro, hydroxyl, -(C₀-C₄-alkanediyl)-COO-(C₀-C₄-alkyl), -CH=CH-COO-(C₀-C₄-alkyl), -(C₀-C₄-alkanediyl)-phenyl, -O-phenyl, -S-phenyl, -NH-(C₁-C₁₈-alkyl), -N(C₁-C₁₈-alkyl)(C₁-C₄-alkyl), -NH-phenyl or -N-(phenyl)(C₁-C₄-alkyl), it being possible for the phenyl constituents to be mono- or di-substituted by C₁-C₄-alkyl, C₁-C₄-alkoxy, amino, nitro, hydroxyl or mixtures thereof, and where in addition two of the radicals R¹, R² and R³, if they are adjacent, can, together with the radical of a fused benzene system, be trimethylene or tetramethylene, each of which may be substituted like the above phenyl constituents.

3. Process according to Claim 2, **characterized in that** aromatic compounds of the formula 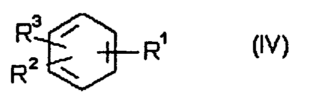 are used, in which
R¹ is as defined in Claim 2,
R² is hydrogen, C₁-C₄-alkyl, C₁-C₄-alkoxy, amino, nitro or hydroxyl, and
R₃ is hydrogen, C₁-C₄-alkyl or C₁-C₄-alkoxy, where in addition the radicals R² and R³, if they are adjacent, may, together with the radical of a fused benzene system, be trimethylene or tetramethylene, each of which can be substituted like the phenyl constituents of R¹.

4. Process according to one or more of Claims 1 to 3, **characterized in that** the hydrogenation is carried out at from 60 to 80°C.

5. Process according to one or more of Claims 1 to 4, **characterized in that** the hydrogenation is carried out at a pressure of from 10 to 400 bar, preferably from 20 to 150 bar.

6. Process according to one or more of Claims 1 to 5, **characterized in that** the process is carried out in the presence of from 150 to 1500%, preferably from 200 to 1000%, of the amount of hydrogen stoichiometrically required for the hydrogenation.

7. Process according to one or more of Claims 1 to 6, **characterized in that** the colloidally dispersed metal as hydrogenation catalyst is a single metal or a metal combination from the group consisting of Pd, Ru, Rh, Pt, Ni, Pd/Ru, Pd/Rh, Ni/Ru and Ni/Rh.

8. Process according to one or more of Claims 1 to 7, **characterized in that** from 1 to 50 atom-% of the hydrogenation-active metal has been replaced by tin in metallic or ionic form.

9. Process according to one or more of Claims 1 to 8, **characterized in that** in the case that a solvent is required to form the first phase with the aromatic compound to be hydrogenated, one from the group consisting of C₅-C₁₂-alkanes, C₅-C₈-cycloalkanes, C₅-C₁₀-alkanols, CO₂, open-chain and cyclic C₄-C₈-ethers and methylene chloride is used in an amount of from 10 to 1000 % by weight, based on the weight of the aromatic compound.

10. Process according to Claim 9, **characterized in that** the amount of solvent used is from 20 to 500% by weight, based on the weight of the aromatic compound.

## Revendications

1. Procédé de préparation de composés carbocycliques à noyau hexagonal de formule (II) par hydrogénation du noyau de composés aromatiques de formule (I) 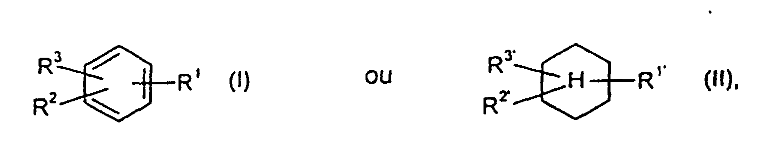 dans lesquelles
R¹, R² et R³ désignent indépendamment l'un de l'autre l'hydrogène, un radical C₁-C₁₈-alkyle à chaîne droite ou ramifiée, un radical C₂-C₁₈-alcényle à chaîne droite ou ramifiée, un radical C₃-C₈-cycloalkyfe, un radical C₁-C₁₈-alcoxy à chaîne droite ou ramifiée, amino, nitro, hydroxy, fluoro, cyano, -(C₀-C₄-alcanediyl)-COO-(C₀-C₄-alkyle), -(C₂-C₄-alcènediyl)-COO-(C₀-C₄-alkyle), C₀-C₄-alcanediyl)-(C₆-C₁₂-aryle), -O-CO-(C₁-C₁₈-alkyle), -O-(C₆-C₁₂-aryle), -CO-(C₁-C₁₈-alkyle), -CO-(C₆-C₁₂-aryle), -S-(C₆-C₁₂-aryle), NH-(C₁-C₁₈-alkyle), - N(C₁-C₁₈-alkyl)(C₁-C₄-alkyle), -NH-(C₆-C₁₂-aryle), -N(C₆-C₁₂-aryl)(C₁-C₄-alkyle) ou -P(X)ₘ(Y)ₙ(Z)_{q}(=O)ᵣ, où les X, Y et Z désignent indépendamment l'un de l'autre un radical phényle ou C₁-C₄-alkyle, et Y et Z peuvent en plus désigner indépendamment l'un de l'autre OR₄ ou NR₄, dans lesquels R₄ désigne un radical phényle, C₁-C₄-alcoxy ou C₁-C₄-alkyle et NR₄ peut aussi représenter un radical pipéridino et Y et Z peuvent aussi désigner ensemble un radical -O-CH₂CH₂-O- et l'expression entre parenthèses représente un oxygène à double liaison, m le chiffre zéro, un ou deux et n, q et r représentent indépendamment l'un de l'autre le chiffre zéro ou un, la somme m+n+q+r correspondant à la valence du phosphore, les éléments aryle pouvant être substitués une ou deux fois par un radical C₁-C₄-alkyle, C₁-C₄-alcoxy, amino, nitro, hydroxy ou différents radicaux parmi eux et deux des résidus R¹, R² et R³, lorsqu'ils sont voisins, pouvant désigner ensemble le résidu d'un système benzène, 9,2-naphtalène ou 2,3-naphtalène condensé, un radical triméthylène, tétraméthylène ou propènediyle qui peuvent être substitués comme les autres composants aryle et
R^{1'}, R^{2'} et R^{3'} adoptent indépendamment l'un de l'autre la signification de R¹, R² et R3 étant entendu qu'à partir des composants oléfiniquement insaturés dans R¹, R² ou R³, les composants saturés correspondants peuvent être formés, à partir d'un radical nitro, un radical amino, à partir de CO, CHOH ou CH₂ et à partir d'un radical cyano, un radical aminométhyle,
**caractérisé en ce que** l'hydrogénation est effectuée à 50 - 180°C et 1 bar - 400 bars dans un système de réaction à partir de deux phases liquides, non miscibles l'une avec l'autre, dans lesquelles de l'hydrogène élémentaire est dispersé, la première phase étant formée par le composé aromatique et, si nécessaire, par un solvant non miscible avec l'eau et la deuxième phase étant formée d'eau, d'au moins un métal actif dans l'hydrogénation et distribué colloïdalement dans l'eau des groupes Ib et Vlll du système périodique des éléments (Mendeleiev) comme catalyseur de l'hydrogénation et adjuvant pour la stabilisation du catalyseur colloïdal et on opère en présence de 100 à 2000 % de la quantité d'hydrogène nécessaire stoechiométriquement.

2. Procédé selon la revendication 1, **caractérisé en ce qu'**on utilise des composés aromatiques de formule 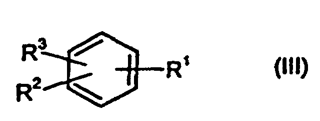 dans laquelle
R¹, R² et R³ désignent indépendamment l'un de l'autre l'hydrogène, un radical C₁-C₁₈-alkyle à chaîne droite ou ramifiée, un radical C₃-, C₅- ou C₆-cycloalkyle, un radical C₁-C₁₈-alcoxy à chaîne droite ou ramifiée, amino, nitro, hydroxy, -(C₀-C₄-alcanediyl)-COO-(C₀-C₄-alkyle), -CH=CH-COO-(C₀-C₄-alkyle), -(C₀-C₄-alcanediyl)-phényle, -O-phényle, -S-phényle, -NH-(C₁-C₁₈-alkyle), -N(C₁-C₁₈-alkyl)(C₁-C₄-alkyle), -NH-phényle ou -N-(phényl)(C₁-C₄-alkyle), les composants phényle pouvant être substitués une ou deux fois par un radical C₁-C₄-alkyle, C₁-C₄-alcoxy, amino, nitro, hydroxy ou différents radicaux parmi eux et deux des résidus R¹, R² et R³, lorsqu'ils sont voisins, pouvant désigner ensemble le résidu d'un système benzène condensé, d'un radical triméthylène ou tétraméthylène qui peuvent être substitués comme les autres composants phényle.

3. Procédé selon la revendication 2, **caractérisé en ce qu'**on utilise des composés aromatiques de formule 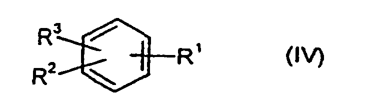 dans laquelle
R¹ a la signification indiquée dans la revendication 2,
R² désigne l'hydrogène, un radical C₁-C₄-alkyle, C₁-C₄-alcoxy, amino, nitro ou hydroxy et
R³ désigne l'hydrogène, un radical C₁-C₄-alkyle ou C₁-C₄-alcoxy, les résidus R² et R³, lorsqu'ils sont voisins, pouvant représenter ensemble le résidu d'un système benzène condensé, d'un radical triméthylène ou tétraméthylène qui peuvent être substitués comme les composants phényle de R¹.

4. Procédé selon l'une ou plusieurs des revendications 1 à 3, **caractérisé en ce que** l'hydrogénation se déroule à une température de 60 à 80°C.

5. Procédé selon l'une ou plusieurs des revendications 1 à 4, **caractérisé en ce que** l'hydrogénation est effectuée à une pression de 10 à 400, de préférence de 20 à 150 bars.

6. Procédé selon l'une ou plusieurs des revendications 1 à 5, **caractérisé en ce qu'**on travaille en présence de 150 à 1.500%, de préférence 200 à 1.000 % de la quantité d'hydrogène nécessaire stoechiométriquement pour l'hydrogénation.

7. Procédé selon l'une ou plusieurs des revendications 1 à 6, **caractérisé en ce qu'**un métal distribué colloïdalement comme catalyseur d'hydrogénation est un métal individuel ou une combinaison de métaux du groupe Pd, Ru, Rh, Pt, Ni, Pd/Ru, Pd/Rh, Ni/Ru et Ni/Rh.

8. Procédé selon l'une ou plusieurs des revendications 1 à 7, **caractérisé en ce que** 1 à 50 % en atome du métal actif dans l'hydrogénation sont remplacés par de l'étain sous forme métallique ou ionique.

9. Procédé selon l'une ou plusieurs des revendications 1 à 8, **caractérisé en ce qu'**au cas où un solvant est nécessaire pour la formation de la première phase avec le composé aromatique à hydrogéner, un composant du groupe des C₅-C₁₂-alcanes, C₅-C₈-cycloalcanes, C₅-C₁₀-alcanols, CO₂ des C₄-C₈-éthers cyclique à chaîne ouverte et du chlorure de méthylène est utilisé dans une quantité de 10 à 1.000 % en poids par rapport au poids du composé aromatique.

10. Procédé selon la revendication 9, **caractérisé en ce que** la quantité du solvant utilisé s'élève à 20 à 500% en poids par rapport au poids du composé aromatique.
